# EUROPEAN PATENT APPLICATION

(11) **EP 3 862 748 A1**
(43) Date of publication of application: **11.08.2021**
(21) Application number: 21154796.3
(22) Date of filing: 02.02.2021
(51) Int. Cl.: G01N 23/04, G01N 23/18, G06T 7/11

(54) **X-RAY INSPECTION DEVICE FOR DETECTING RESIDUAL BONES IN CHICKEN BREAST MEAT**

(30) Priority: 05.02.2020 JP 2020018152
(71) Applicant: ISHIDA CO., Ltd., Kyoto-shi, Kyoto 606-8392 (JP)
(72) Inventor: KUDO, Daisuke, Ritto-shi, Shiga 520-3026 (JP); IKEDA, Jun, Ritto-shi, Shiga 520-3026 (JP); YAMAMOTO, Yoichi, Ritto-shi, Shiga 520-3026 (JP); TODORI, Michihiko, Ritto-shi, Shiga 520-3026 (JP); SUGIMOTO, Kazuyuki, Ritto-shi, Shiga 520-3026 (JP)
(74) Representative: Global IP Europe Patentanwaltskanzlei

(57) **Abstract**

Provided is an X-ray inspection device including: an image generation unit configured to generate an X-ray image from a detection result of X-rays transmitted through an inspection object; and a control unit configured to process the X-ray image generated by the image generation unit. The control unit virtually divides the inspection object projected on the X-ray image for each of regions of the inspection object, inspects the X-ray image for every region, and records the inspection result for the every region in association with region information relating to each of the regions.

## Description

### TECHNICAL FIELD

An aspect of the present invention relates to an X-ray inspection device.

### BACKGROUND

As an X-ray inspection device in the related art, for example, a device described in International Publication WO 2006/001107 A is known. The X-ray inspection device described in International Publication WO 2006/001107 A includes an X-ray irradiation unit that irradiates an article (inspection object) with X-rays, and an X-ray detection unit that detects X-rays. Image processing is performed with respect to an X-ray image generated on the basis of a detection result of the X-rays to perform inspection on the article.

### SUMMARY

Inspection for foreign matters mixed in an inspection object (for example, inspection for residual bones of chicken) is performed with the above-described X-ray inspection device. In the inspection for the foreign matters, for example, it may be required to easily understand that the foreign matters exist in which region of the inspection object so as to raise efficiency when an operator removes the foreign matters on the basis of the inspection result.

An object of an aspect of the invention is to provide an X-ray inspection device capable of easily understanding that foreign matters exist in which region of an inspection object.

According to an aspect of the invention, there is provided an X-ray inspection device including: an image generation unit configured to generate an X-ray image from a detection result of X-rays transmitted through an inspection object; and a control unit configured to process the X-ray image generated by the image generation unit. The control unit is further configured to virtually divide an image of the inspection object projected on the X-ray image into virtual regions which correspond to physical regions of the inspection object, inspect the X-ray image for each virtual region, and record the inspection result for each virtual region in association with region information relating to each of the physical regions of the inspection object.

In the X-ray inspection device, the inspection result for each of regions in the inspection object (hereinafter, also referred to as "inspection result for each of regions") is stored in association with the region information. Accordingly, when using the stored inspection result for each of regions, it is possible to easily understand that foreign matters exist in which region of the inspection object.

In the X-ray inspection device according to the aspect of the invention, each virtual region may be set for each of portions that are classified by a feature of the inspection object. In this case, each region of the inspection object can be specifically set.

In the X-ray inspection device according to the aspect of the invention, the control unit may be configured to aggregate the inspection result for each virtual region with respect to a plurality of the inspection objects to generate statistical data. According to this, the generated statistical data can be used, for example, in adjustment of a device that removes foreign matters from the inspection object.

The X-ray inspection device according to the aspect of the invention may further include an input unit configured to receive an input of worker information relating to a worker. The control unit may be configured to record the inspection result for each virtual region further in association with the worker information received by the input unit. According to this, a worker who removes foreign matters from the inspection object can recognize a region that tends to be left, and can perform work with focus given to the region.

In the X-ray inspection device according to the aspect of the invention, the control unit may be configured to record at least one of a size of detected foreign matters, the number of the foreign matters, the thickness of the foreign matters, and a size of the inspection object for each virtual region as a result of the inspection. According to this, an inspection result can be obtained for each of specific regions.

In the X-ray inspection device according to the aspect of the invention, the inspection object may be chicken. In this case, according to the X-ray inspection device, inspection for residual bones of the chicken can be carried out. The inspection result for each of regions can be used for bone removal work for removing bone from the chicken.

The X-ray inspection device according to the aspect of the invention may further include a display unit that is configured to display the inspection result for each virtual region which is recorded by the control unit. In this case, since the inspection result for each of regions is shown by the display unit to a worker who removes foreign matters from the inspection object, work efficiency can be raised.

### BRIEF DESCRIPTION OF THE DRAWINGS

FIG. 1 is a configuration diagram of an X-ray inspection device according to an embodiment.
FIG. 2 is a configuration diagram of the inside of a shield box illustrated in FIG. 1.
FIGS. 3A and 3B are views illustrating an X-ray image.
FIG. 4 is a view illustrating each region of breast meat.
FIG. 5 is a view illustrating an image for description of region division processing.
FIGS. 6A and 6B are views illustrating an image for description of the region division processing.
FIGS. 7A and 7B are views illustrating an image for description of the region division processing.
FIGS. 8A and 8B are views illustrating an image for description of the region division processing.
FIGS. 9A, 9B, 9C, and 9D are views illustrating a display example of a display operation unit.

### DETAILED DESCRIPTION

Hereinafter, an embodiment will be described in detail with reference to the accompanying drawings. Note that, in description of the drawings, the same reference numeral will be given to the same or equivalent element, and redundant description thereof will be omitted.

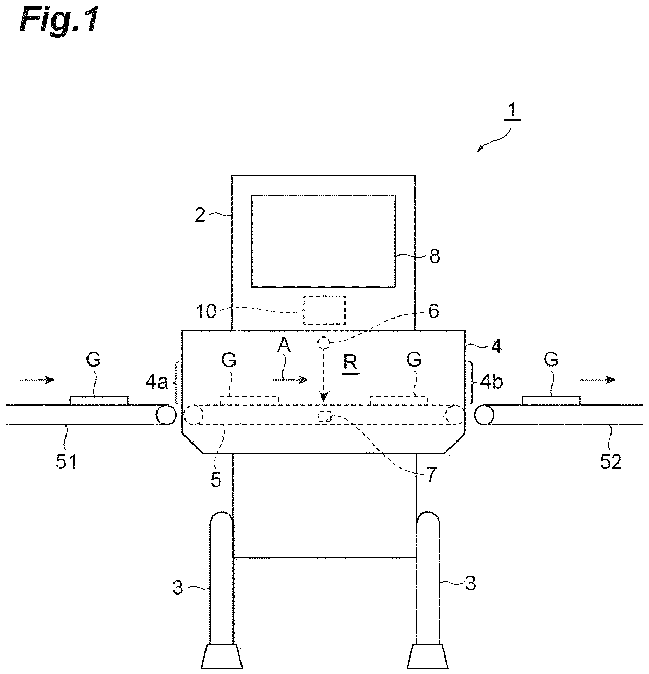

As illustrated in FIG. 1, an X-ray inspection device 1 includes a device main body 2, a supporting leg 3, a shield box 4, a conveyance unit 5, an X-ray irradiation unit 6, an X-ray detection unit 7, a display operation unit 8, and a control device 10.

The X-ray inspection device 1 generates an X-ray image of an article G while conveying the article (inspection object) G, and carries out inspection on the article G (for example, inspection for the number of storage, inspection for mixing-in of foreign matters, inspection for a defect article, inspection for crack and chipping, and the like) on the basis of the X-ray image. The article G before inspection is carried into the X-ray inspection device 1 by a carrying-in conveyor 51. The article G after inspection is carried out from the X-ray inspection device 1 by a carrying-out conveyor 52. An article G that is determined as a defective article by the X-ray inspection device 1 is sorted out of a production line by a sorting device (not illustrated) disposed downstream the carrying-out conveyor 52. An article G that is determined as a good article by the X-ray inspection device 1 passes through the sorting device as is. For example, the article G is chicken (particularly, breast meat). All objects are applicable as the article G without particular limitation. The article G may be another meat, or may be fish.

The device main body 2 accommodates the control device 10 and the like. The supporting leg 3 supports the device main body 2. The shield box 4 is provided in the device main body 2. The shield box 4 prevents leakage of X-rays to the outside. An inspection region R in which inspection on the article G with X-rays is carried out is provided inside the shield box 4. A carrying-in port 4a and a carrying-out port 4b are provided in the shield box 4. The article G before inspection is carried into the inspection region R from the carrying-in conveyor 51 through the carrying-in port 4a. The article G after inspection is carried out from the inspection region R to the carrying-out conveyor 52 through the carrying-out port 4b. An X-ray shield curtain (not illustrated) that prevents leakage of X-rays is provided in the carrying-in port 4a and the carrying-out port 4b.

The conveyance unit 5 is disposed inside the shield box 4. The conveyance unit 5 conveys the article G from the carrying-in port 4a to the carrying-out port 4b through the inspection region R along a conveyance direction A. For example, the conveyance unit 5 is a belt conveyor that extends between the carrying-in port 4a and the carrying-out port 4b.

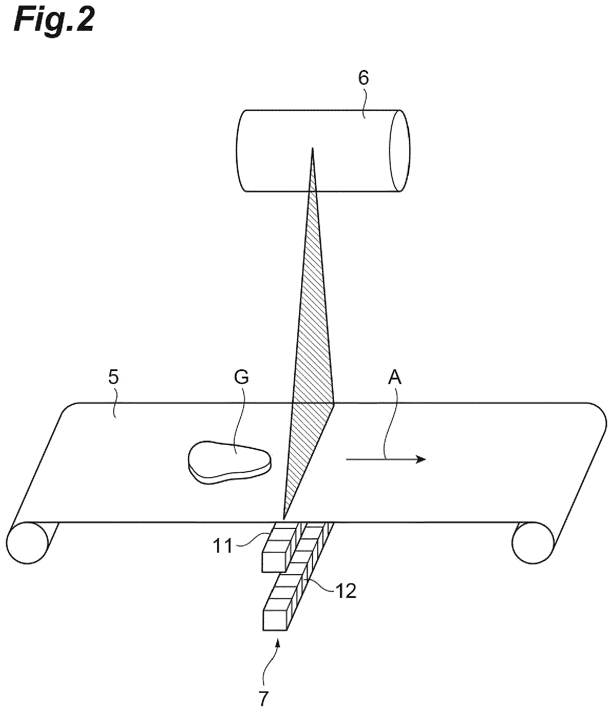

As illustrated in FIG. 1 and FIG. 2, the X-ray irradiation unit 6 is disposed inside the shield box 4. The X-ray irradiation unit 6 irradiates the article G that is conveyed by the conveyance unit 5 with X-rays. For example, the X-ray irradiation unit 6 includes an X-ray tube that emits X-rays, and a collimator that expands the X-rays emitted from the X-ray tube in a fan shape in a plane orthogonal to the conveyance direction A.

The X-ray detection unit 7 is disposed inside the shield box 4. The X-ray detection unit 7 includes a first line sensor 11 and a second line sensor 12. The first line sensor 11 and the second line sensor 12 are constituted by X-ray detection elements which are one-dimensionally arranged along a horizontal direction orthogonal to the conveyance direction A. The first line sensor 11 detects X-rays of a low energy band (first energy band) which are transmitted through the article G and a conveyance belt of the conveyance unit 5. The second line sensor 12 detects X-rays of a high energy band (second energy band) which are transmitted through the article G, the conveyance belt of the conveyance unit 5, and the first line sensor 11.

As illustrated in FIG. 1, the display operation unit 8 is provided in the device main body 2. The display operation unit 8 displays various pieces of information and receives input of various conditions. For example, the display operation unit 8 is a liquid crystal display, and displays an operation screen as a touch panel. In this case, a worker can input the various conditions through the display operation unit 8. The display operation unit 8 can input worker information relating to the worker. As to be described later, the display operation unit 8 displays an inspection result for each of regions which is recorded by the control device 10 and the like.

The control device 10 is disposed inside the device main body 2. The control device 10 controls operations of respective parts of the X-ray inspection device 1. The control device 10 is constituted by a central processing unit (CPU), a read only memory (ROM), a random access memory (RAM), and the like. The detection result of the X-rays of the low energy band is input to the control device 10 from the first line sensor 11 (refer to FIG. 2) of the X-ray detection unit 7, and the detection result of the X-rays of the high energy band is input to the control device 10 from the second line sensor 12 (refer to FIG. 2) of the X-ray detection unit 7.

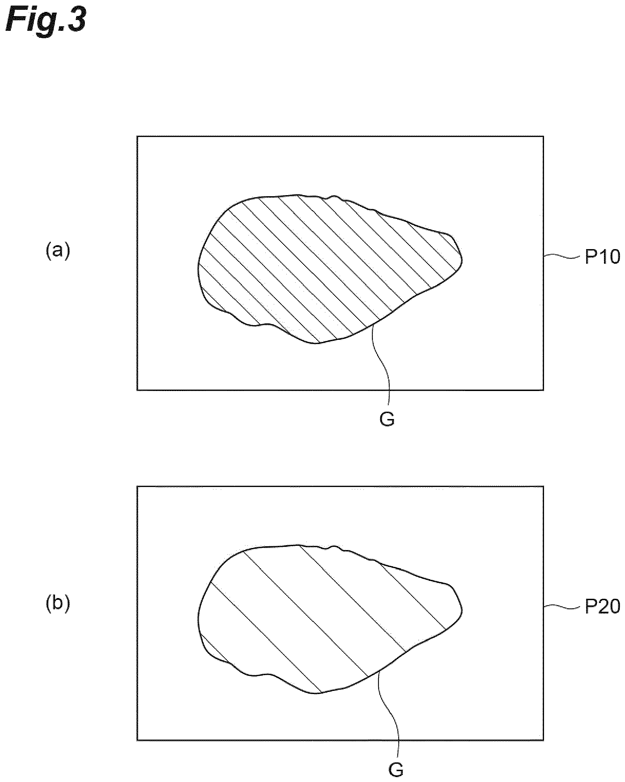

The control device 10 generates an X-ray image on the basis of the detection results by the first line sensor 11 and the second line sensor 12. As illustrated in FIG. 3A, the control device 10 generates an X-ray image P10 on the basis of the detection result of the X-rays of the low energy band by the first line sensor 11. The X-ray image P10 has relatively high contrast, and is dark as a whole. As illustrated in FIG. 3B, the control device 10 generates an X-ray image P20 on the basis of the detection result of the X-rays of the high energy band by the second line sensor 12. The X-ray image P20 has relatively low contrast, and is bright as a whole.

The control device 10 processes the X-ray images P10 and P20 which are generated. Specifically, the control device 10 carries out region division processing of virtually dividing the article G projected on the X-ray images P10 and P20 for each of regions of the article G, inspection processing of inspecting the X-ray images P10 and P20 for each of regions, and recording processing of recording the inspection result for each of regions in association with region information relating to each of the regions. Hereinafter, the region division processing, the inspection processing, and the recording processing will be described. An image of the article G (the breast meat G) may be simply referred to as the "article G" or the "breast meat G" below.

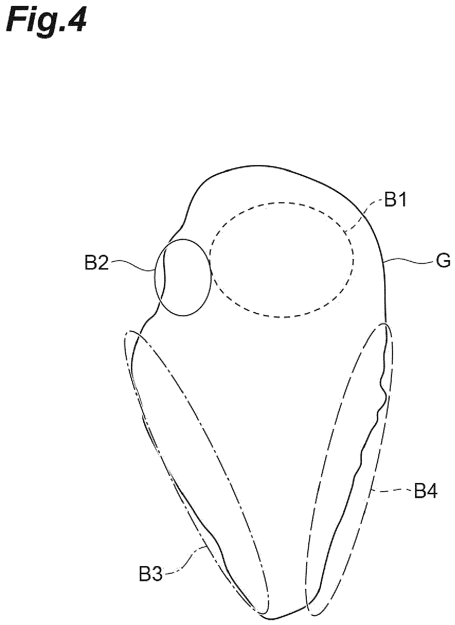

With regard to the region division processing, description will be given of a case where the article G is chicken (breast meat) as an example. The region can be set for each of portions classified according to a feature of the article G. Examples of the feature include a shape (a straight line, a curved line, a rectangle, a triangle, or the like), the thickness, and a position (an end portion or a central portion) of the article G. As illustrated in FIG. 4, as the region, the article G (hereinafter, also referred to as "breast meat G") as the breast meat includes portions classified by the feature of the article G, that is, a clavicle portion B1, a wing stick portion B2, a triquetral bone portion B3, and a sternum portion B4. Since the breast meat G has a feature in a shape, even in a case where a direction when being conveyed by the conveyance unit 5 is not constant direction, the respective regions can be specified.

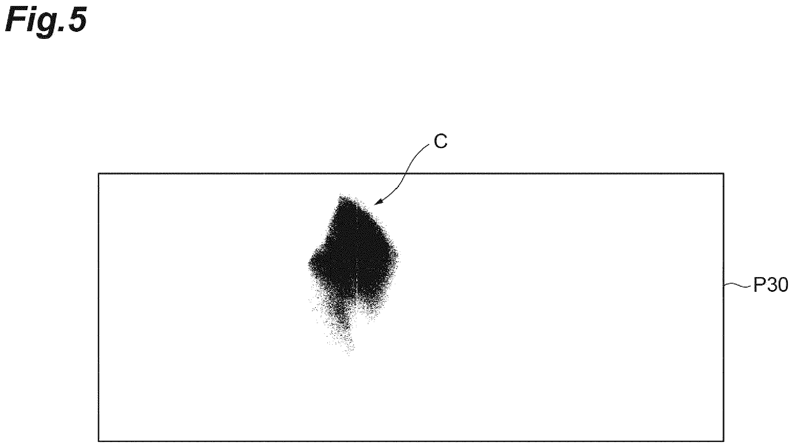

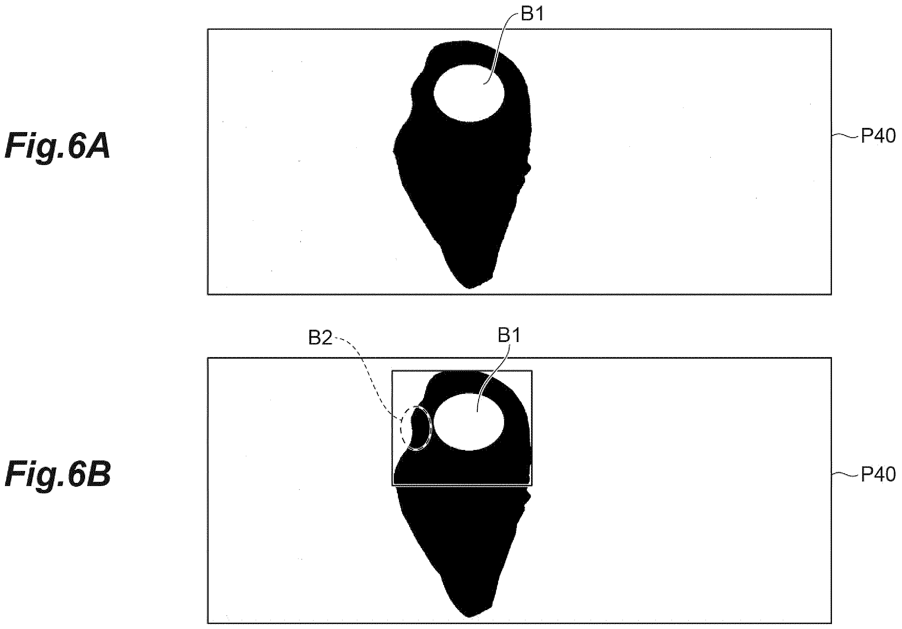

In the region division processing, first, the clavicle portion B1 is specified. For example, the X-ray images P10 and P20 are binarized from a dark side of an entire histogram by a value of a predetermined percentage to obtain an image P30 illustrated in FIG. 5. According to this, a region C having the greatest thickness is obtained. As illustrated in FIG. 6A, the X-ray images P10 and P20 are subjecting to image processing to obtain an image P40 binarized so that the breast meat G becomes dark. In addition, in the image P40, a portion corresponding to the obtained region C is specified as the clavicle portion B1.

Next, the wing stick portion B2 is specified. For example, as illustrated in FIG. 6B, in the image P40, a rectangle D is drawn at the periphery of the clavicle portion B1, and a side where a difference (gap) between the rectangle D and the breast meat G is large is determined as "non-smooth side". A region that is located at the periphery of the clavicle portion B1 and on the "non-smooth side" is specified as the wing stick portion B2. Note that, a circle may be drawn on the image P40 instead of the rectangle D.

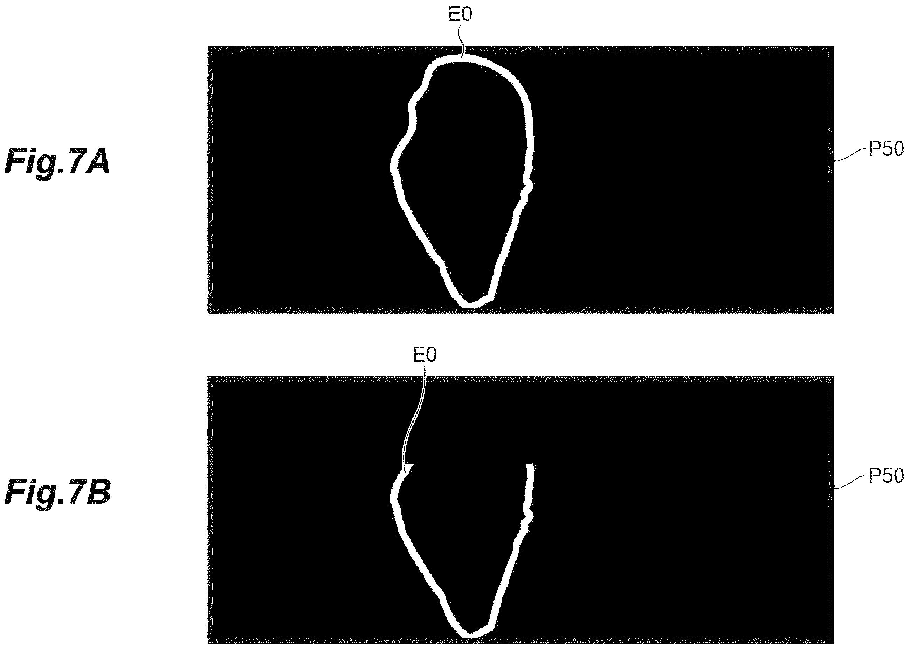

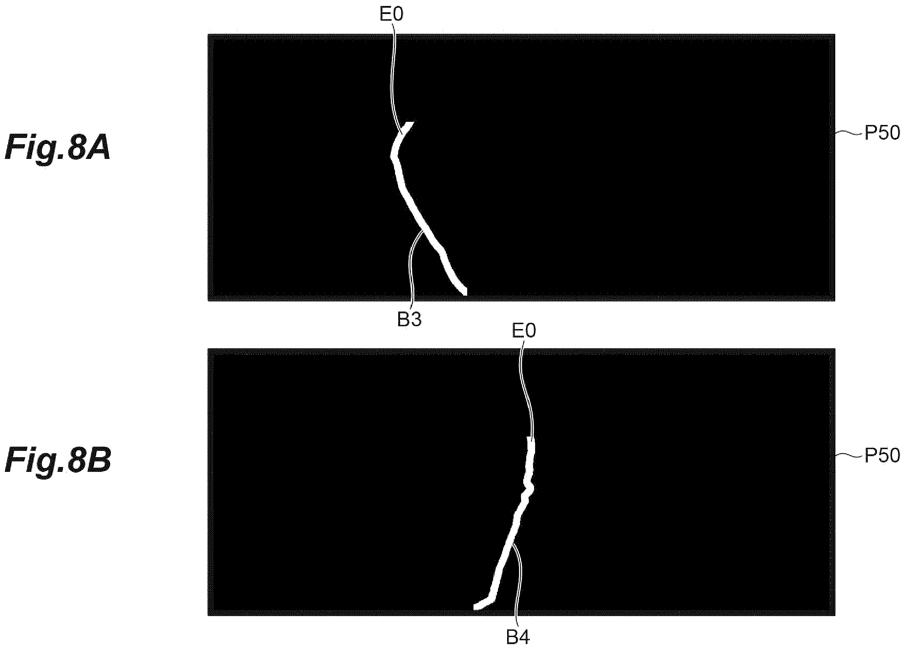

Next, a peripheral edge of the breast meat G is obtained to specify the triquetral bone portion B3 and the sternum portion B4. For example, as illustrated in FIG. 7A, the X-ray images P10 and P20 are subjected to image processing to obtain an image P50 binarized so that a peripheral edge E0 of the breast meat G becomes white. An inflection point of a "non-smooth side" in the peripheral edge E0 is obtained. As illustrated in FIG. 7B, in the peripheral edge E0, a portion on the wing stick portion B2 side is erased from a position advanced toward the wing stick portion B2 side to a certain extent from the inflection point. As illustrated in FIG. 8A, in the peripheral edge E0 from which the portion on the wing stick portion B2 side is erased, a region corresponding to a "non-smooth side" is specified as the triquetral bone portion B3. As illustrated in FIG. 8B, in the peripheral edge E0 from which a portion on the wing stick portion B2 side is erased, a region corresponding to a "smooth side" is specified as the sternum portion B4. As a result, the breast meat G projected on the X-ray images P10 and P20 are virtually divided into the clavicle portion B1, the wing stick portion B2, the triquetral bone portion B3, and the sternum portion B4.

Next, inspection processing will be described. In the inspection processing, inspection for foreign matters is carried out for each of regions by carrying out first image processing and second image processing with respect to the X-ray images P10 and P20 by using a plurality of image processing algorithms. In a case where the article G is the breast meat (breast meat G), inspection for foreign matters relating to residual bones is carried out for each of the clavicle portions B1, the wing stick portion B2, the triquetral bone portion B3, and the sternum portion B4.

The image processing algorithms are a type indicating a processing procedure of the image processing carried out with respect to the X-ray images P10 and P20. The image processing algorithms are constituted by one image processing filter, or a combination of a plurality of image processing filters. The plurality of image processing algorithms can be acquired from the outside through a network such as the Internet. In addition, the plurality of image processing algorithms can also be acquired from an external storage medium such as a USB memory and a removable hard disk. At least one or more among the plurality of image processing algorithms can be automatically generated from a plurality of image processing filters on the basis of specifications of the X-ray inspection device 1, inspection conditions, or the like by employing genetic algorithms (GA) that is a method applying a mechanism of heredity and evolution in the biological world. At least a part of the plurality of image processing algorithms can also be appropriately set by a worker through the display operation unit 8. The first image processing and the second image processing may employ various kinds of known image processing without particular limitation.

The inspection for foreign matters which is carried out for each of regions can be carried out by using two image processing algorithms. Specifically, the first image processing is carried out with respect to the X-ray images P10 and P20 by using a first image processing algorithm among a plurality of image processing algorithms to determine whether or not foreign matters are contained in the article G. In combination with this, the second image processing is carried out with respect to the X-ray images P20 (X-ray image P10) by using a second image processing algorithm among the plurality of image processing algorithms to determine whether or not foreign matters are contained in the article G. In addition, in a case where foreign matters common to determination results of the first image processing and the second image processing are contained in an arbitrary region of the article G, it is determined that the foreign matters are contained in the region. Note that, since the mixed-in foreign matters have a feature for each of regions, an image processing algorithm corresponding to the region may be used in inspection for foreign matters with respect to each region.

Next, recording processing will be described. In the recording processing, at least any one of the size of foreign matters detected, the number of the foreign matters, the thickness of the foreign matters, and the size of the article G is recorded for each of regions as an inspection result. In the recording processing, the inspection result for each of regions is recorded further in association with worker information that is input by the display operation unit 8. For example, the worker information is information relating to a worker who is in charge of bone removal work of the breast meat G. For example, the worker information may be at least any one of the name of the worker, an identification number, and the like without particular limitation. In the recording processing, the inspection result for each of regions is aggregated with respect to a plurality of the articles G, and statistical data is generated.

The control device 10 causes the display operation unit 8 to display information recorded in the recording processing, for example, in correspondence with an operation by a worker. The inspection result for each of regions which is associated with the region information may be displayed on the display operation unit 8. At least any one of the size of foreign matters detected, the number of the foreign matters, the thickness of the foreign matters, and the size of the article G may be displayed on the display operation unit 8 for each of regions. The inspection result for each of regions may be displayed on the display operation unit 8 further in association with the worker information. The statistical data obtained by aggregating the inspection result for each of regions with respect to the plurality of articles G may be displayed on the display operation unit 8.

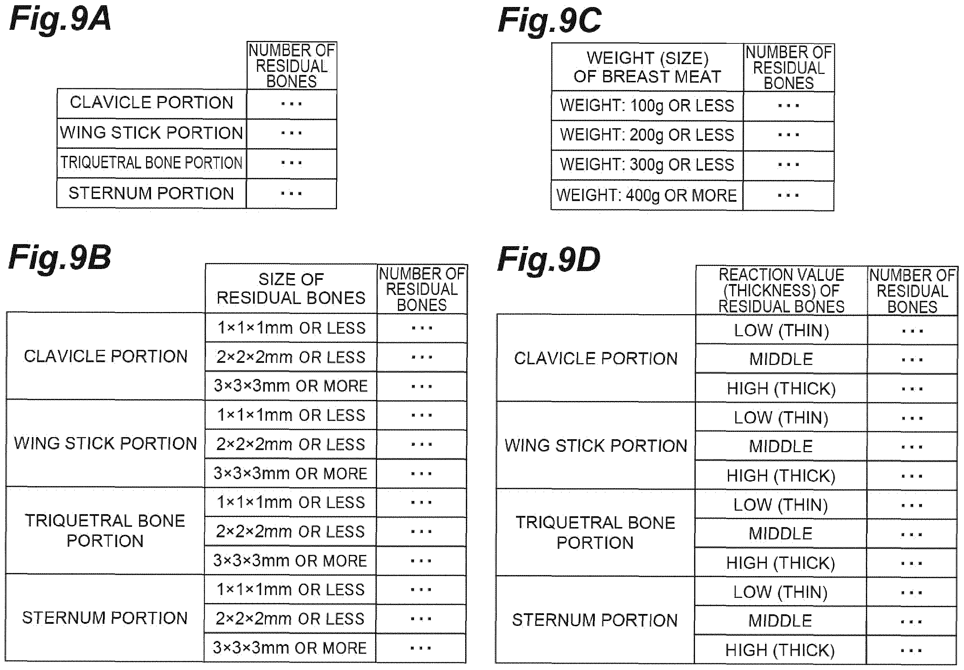

As an example, the display operation unit 8 can display a residual bone inspection result obtained by counting the number of residual bones with respect to an arbitrary breast meat G for each of the clavicle portions B1, the wing stick portion B2, the triquetral bone portion B3, and the sternum portion B4 (refer to FIG. 9A). The display operation unit 8 can display a residual bone inspection result obtained by counting the number of residual bones with respect to an arbitrary breast meat G in classification into the weight (size) of the residual bones for each of the clavicle portions B1, the wing stick portion B2, the triquetral bone portion B3, and the sternum portion B4 (refer to FIG. 9B).

In addition, as an example, the display operation unit 8 can display a residual bone inspection result obtained by counting the number of residual bones with respect to a plurality of the breast meat G in classification into the size of the breast meat G (refer to FIG. 9C). The display operation unit 8 can display a residual bone inspection result obtained by counting the number of residual bones with respect to an arbitrary breast meat G in classification into a reaction value (thickness) of the residual bones for each of the clavicle portions B1, the wing stick portion B2, the triquetral bone portion B3, and the sternum portion B4 (refer to FIG. 9D). The reaction value of the residual bones is a value detected in the above-described inspection processing, and can be classified into "low", "middle", and "high". When the reaction value is "low", the thickness is small, when the reaction value is "high", the thickness is large, and when the reaction value is "middle", the thickness is middle.

As described above, in the X-ray inspection device 1, the inspection result for each of regions which is an inspection result for each of regions in the article G is stored in association with the region information. Accordingly, when using the stored inspection result for each of regions, it is possible to easily understand that foreign matters exist in which region of the article G. As a result, foreign matter detection performance can be raised.

In the X-ray inspection device, the region is set for each of portions that is classified by a feature of the article G. In this case, each region of the article G can be specifically set.

In the X-ray inspection device 1, the inspection result for each of regions is aggregated with respect to the plurality of articles G, and statistical data is generated. According to this, the generated statistical data can be used in adjustment of a device (for example, an upstream chicken disassembly device or the like) that removes foreign matters from the article G. Accuracy of a device that removes foreign matters from the article G is raised, and foreign matter mixing-in in the article G can be reduced.

The X-ray inspection device 1 includes the display operation unit 8 capable of inputting worker information. In the X-ray inspection device 1, the inspection result for each of regions is recorded further in association with the input worker information. According to this, the worker can recognize a region that tends to be left, and can perform work with focus given to the region.

In the X-ray inspection device 1, at least any one of the size of foreign matters detected, the number of the foreign matters, the thickness of the foreign matters, and the size of the article G is recorded as the inspection result for each of regions. According to this, a specific inspection result for each of regions can be obtained.

In the X-ray inspection device 1, the article G is chicken. In this case, according to the X-ray inspection device 1, inspection for residual bones of the chicken can be carried out, and the inspection result for each of regions can be used for bone removal work for removing bone from the chicken. Particularly, in the breast meat G, since a position where bones remain is limited in many cases, the X-ray inspection device 1 is effective.

In the X-ray inspection device 1, the recorded inspection result for each of regions is displayed on the display operation unit 8. According to this, the inspection result for each of regions is shown to a worker, and the efficiency of the work (for example, bone removal work) can be raised.

Hereinbefore, the embodiment has been described, but the aspect of the invention is not necessarily limited to the embodiment, and various modifications can be made within a range not departing from the gist.

In the embodiment, description has been given of an aspect in which the X-ray detection unit 7 includes the first line sensor 11 and the second line sensor 12 as an example. However, the X-ray detection unit may include at least one line sensor. In this case, the control device 10 may carry out inspection processing by using a plurality of different image processing algorithms on the basis of one X-ray image.

In the embodiment, in a case where foreign matters common to determination results of the first image processing and the second image processing are contained in the article G, it is determined that foreign matters are contained. However, a determination image may be further generated on the basis of the determination image generated by the first image processing and the determination image generated by the second image processing, and a third threshold value may be applied to the further generated determination image to determine whether or not foreign matters are contained in the article G.

In the embodiment, description has been given of an aspect in which the X-ray inspection device 1 includes the conveyance unit 5 as an example. However, the X-ray inspection device 1 may not include the conveyance unit 5. As described above, the control device 10 constitutes an image generation unit and a control unit, and the display operation unit 8 constitutes an input unit and a display unit. In the embodiment, a region of the article G is set for each of portions (the clavicle portion B1, the wing stick portion B2, the triquetral bone portion B3, or the sternum portion B4) of the article G, but there is no limitation thereto. The region of the article G may be various regions which are appropriately set in the article G.

Various materials and shapes are applicable to respective configurations in the above-described embodiment and modification example without limitation to the above-described materials and shapes. The respective configurations in the above-described embodiment and modification example are arbitrarily applicable to respective configuration of another embodiment or modification example. A part of the respective configurations in the above-described embodiment or modification example can be appropriately omitted within a range not departing from the gist of the aspect of the invention.

According to the aspect of the invention, it is possible to provide an X-ray inspection device capable of easily understanding that foreign matters exist in which region of the inspection object.

## Claims

1. An X-ray inspection device comprising:
an image generation unit configured to generate an X-ray image from a detection result of X-rays transmitted through an inspection object; and
a control unit configured to process the X-ray image generated by the image generation unit,
wherein the control unit is further configured to:
virtually divide an image of the inspection object projected on the X-ray image into virtual regions which correspond to physical regions of the inspection object,
inspect the X-ray image for each virtual region, and
record the inspection result for each virtual region in association with region information relating to each of the physical regions of the inspection object.

2. The X-ray inspection device according to claim 1,
wherein each virtual region is set for each of portions that are classified by a feature of the inspection object.

3. The X-ray inspection device according to claim 1 or 2,
wherein the control unit is configured to aggregate the inspection result for each virtual region with respect to a plurality of the inspection objects to generate statistical data.

4. The X-ray inspection device according to any one of claims 1 to 3, further comprising:
an input unit configured to receive an input of worker information relating to a worker,
wherein the control unit is configured to record the inspection result for each virtual region further in association with the worker information received by the input unit.

5. The X-ray inspection device according to any one of claims 1 to 4,
wherein the control unit is configured to record at least one of a size of detected foreign matters, the number of the foreign matters, the thickness of the foreign matters, and a size of the inspection object for each virtual region as a result of the inspection.

6. The X-ray inspection device according to any one of claims 1 to 5,
wherein the inspection object is chicken.

7. The X-ray inspection device according to any one of claims 1 to 6, further comprising:
a display unit configured to display the inspection result for each virtual region which is recorded by the control unit.
